# EUROPEAN PATENT APPLICATION

(11) **EP 2 777 655 A2**
(43) Date of publication of application: **17.09.2014**
(21) Application number: 14158435.9
(22) Date of filing: 07.03.2014
(51) Int. Cl.: A61F 5/01

(54) **Extension limiting strap for a hip brace**

(30) Priority: 15.03.2013 US 201313831676
(71) Applicant: Breg, Inc., Carlsbad, CA 92010 (US)
(72) Inventor: Hollister, Matthew. T, Encinitas, CA 92024 (US); Christoforetti, John, Pittsburgh, PA 15238 (US)
(74) Representative: Meldrum, David James

(57) **Abstract**

A hip brace is provided having a waist belt, waist cuff, longitudinal support assembly and hip joint extension limiting strap. The waist belt is secured around the waist of a wearer and the waist cuff and longitudinal support assembly are positioned on the side of the body adjacent to the affected hip joint. The waist cuff is attached to the waist belt above the hip joint and the longitudinal support assembly extends from the waist cuff past the hip joint along the length of the thigh and is hinged in correspondence with the hip joint. The extension limiting strap extends posteriorly from a lateral anchor position on the waist cuff to a posterior anchor position on the waist belt and prevents undesirable migration of the waist cuff during hip joint extension.

## Description

### BACKGROUND

The present disclosure relates generally to an orthopedic brace, and more particularly to an orthopedic brace for reinforcing and stabilizing the hip.

Orthopedic braces embody a broad range of apparatuses, each having the common purpose of supporting and/or stabilizing a skeletal joint when worn on the body of a user. The orthopedic brace may serve either a preventative role or a remedial role. In a preventative role, the brace provides added support and stability to a healthy skeletal joint, thereby reducing the risk of injury when the joint is subjected to undue stress. In a remedial role, the brace supports and stabilizes a skeletal joint which has been weakened by injury or other infirmity, thereby reinforcing the joint and reducing the risk of further injury while the joint is rehabilitated. One conventional type of orthopedic brace is a hip brace which is specifically configured for supporting and stabilizing the hip joint.

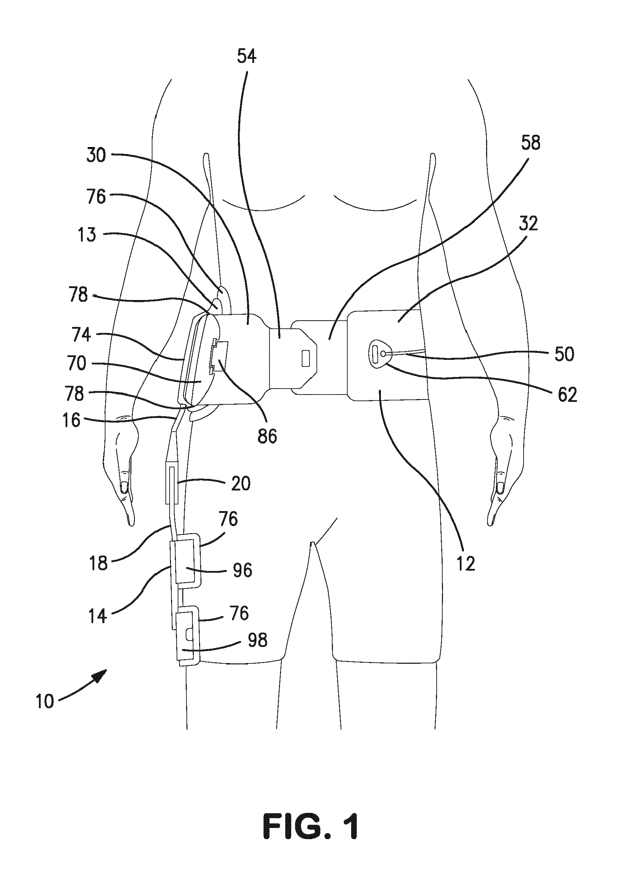

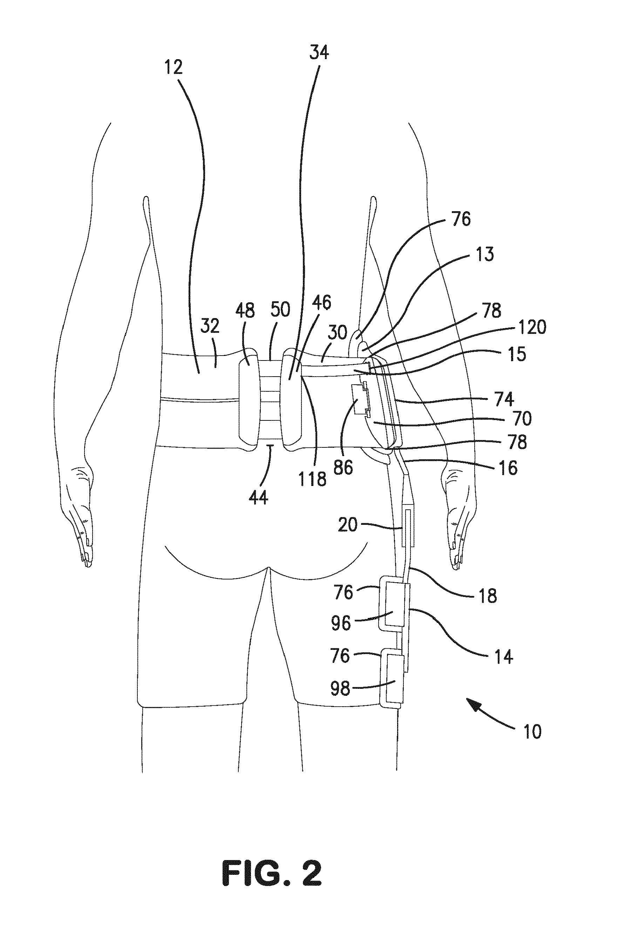

An example conventional hip brace is disclosed in U.S. Patent No. 4,481,941 to Rolfes which is incorporated herein by reference. The hip brace (20) of Rolfes includes waist engaging means (24), upper and lower extension members (30, 32), a hinge (28) and thigh engaging means (26) as shown in FIGS. 1 and 2 of Rolfes. A belt (50) and a plate (64) attached thereto are part of the waist engaging means (24) and worn around the waist of the user. The hinge rotatably connects the upper and lower extension members to one another, which in combination extend downward from the lateral side of the belt across the affected hip joint and along the lateral side of the thigh below the hip joint. The upper extension member is positioned adjacent to the lateral side of the waist and is secured in position by attachment to the plate which is in turn attached to the belt. The lower support member is positioned adjacent to the lateral side of the thigh and is secured in position by a plurality of releasable straps (116) which are included in the thigh engaging means. The hinge is positioned between the upper and lower extension members adjacent to the hip joint and allows rotational displacement of the extension members relative to one another in correspondence with flexion and extension of the hip joint. An important feature of the hinge are the limiting screws (46, 48) which the user positions on the hinge as shown in FIG. 6 to selectively set rotation limits for the hinge in either direction of rotation. It is apparent that by limiting rotation of the hinge in this manner, the hip brace correspondingly limits the range of motion of the hip joint on which it is mounted in either the flexion or extension direction.

One problem presented by conventional hip braces of the type described above is that the force the upper extension member applies to the plate as the hip joint moves to extension tends to undesirably rotate the plate forward and actually permits the hip joint to rotate in extension beyond the extension rotation limit setting of the hinge. As such, conventional hip braces often lack the ability to limit extension of the hip joint with a degree of precision desirable for effective function of the hip brace in either a preventative or remedial role.

### SUMMARY

The present teachings recognize the drawbacks of known hip braces and propose an improved hip brace which limits the extension of the hip joint on which it is mounted with a high degree of precision.

Particular aspects and embodiments are recited in the appended claims.

Viewed from one aspect, there can be provided a hip brace exhibiting function which limits the extension of the hip joint on which it is mounted with a high degree of precision. In some examples, there can be provided a hip brace which has effective means for precisely limiting the extension of the hip joint on which the hip brace is mounted.

Viewed from another aspect, there can be provided a hip brace comprising a waist belt, waist cuff, longitudinal support assembly and extension limiting strap. The waist belt is wrappable around a waist of a wearer of the hip brace. The waist belt has a circumference including a first lateral side, a second lateral side, a posterior side and an anterior side. The waist cuff, which has an anterior side and a posterior side, is attached to the first lateral side of the waist belt. The waist cuff is substantially less pliant than the waist belt. The longitudinal support assembly is attached to the waist cuff and extends downwardly away from the waist cuff. The longitudinal support assembly includes a first support member having a first end attached to the waist cuff and a second end. The longitudinal support assembly further includes a rotational hinge attached to the second end of the first support member and aligned with a hip joint of the wearer adjacent to the first lateral side of the waist belt. The longitudinal support assembly still further includes a second support member having a first end. The hinge is attached to the first end of the second member, thereby rotatably connecting the first and second members.

The extension limiting strap is attached to the waist cuff at a first anchor point and is attached to the waist belt at a second anchor point positioned an anchoring interval away from the first anchor point in a posterior direction. As such, the extension limiting strap extends between the first and second anchor points. The extension limiting strap relaxes when the hinge travels in a hip flexion direction and tensions when the hinge travels in a hip extension direction to apply a resistant counter-force to the waist cuff in a first force direction in response to a moment of force applied to the waist cuff by the longitudinal support assembly in a second force direction which is substantially opposite the first force direction.

In accordance with one embodiment, the first anchor point is proximal to a posterior upper corner of the waist cuff. In accordance with another embodiment, the anchoring interval is at least about one quarter the circumference of the waist belt. In accordance with yet another embodiment, the waist cuff is rigid and the extension limiting strap is pliant. In accordance with another embodiment, the first force direction is anterior and the second force direction is posterior.

Viewed from a further aspect, there can be provided a hip brace comprising a waist belt, waist cuff, longitudinal support assembly and extension limiting strap. The waist belt is wrappable around a waist of a wearer of the hip brace. The waist belt has a circumference including a first lateral side, a second lateral side, a posterior side and an anterior side. The waist cuff is attached to the waist belt and is substantially more rigid than the waist belt. The longitudinal support assembly is attached to the waist cuff and extends away from the waist cuff. The extension limiting strap is attached to the waist cuff at a first anchor point and is attached to the waist belt at a second anchor point positioned an anchoring interval away from the first anchor point in a posterior direction. As such the extension limiting strap extends between the first and second anchor points. The extension limiting strap relaxes when the longitudinal support assembly travels in a hip flexion direction and tensions when the longitudinal support assembly travels in a hip extension direction to apply a resistant counter-force to the waist cuff in a first force direction in response to a moment of force applied to the waist cuff by the longitudinal support assembly in a second force direction which is substantially opposite the first force direction.

Viewed from another aspect, there can be provided a hip brace comprising a waist belt, waist cuff, longitudinal support assembly and extension limiting strap. The waist belt is wrappable around a waist of a wearer of the hip brace. The waist belt has a circumference including a first lateral side, a second lateral side, a posterior side and an anterior side. The waist cuff, which has an anterior side and a posterior side, is attached to the first lateral side of the waist belt. The waist cuff is substantially more rigid than the waist belt. The longitudinal support assembly is attached to the waist cuff and extends downwardly away from the waist cuff. The longitudinal support assembly includes a first support member having a first end attached to the waist cuff and a second end. The longitudinal support assembly further includes a rotational hinge attached to the second end of the first support member and aligned with a hip joint of the wearer adjacent to the first lateral side of the waist belt. The longitudinal support assembly still further includes a second support member having a first end. The hinge is attached to the first end of the second member, thereby rotatably connecting the first and second members. The extension limiting strap is attached to the waist cuff at a first anchor point and is attached to the waist belt at a second anchor point positioned an anchoring interval away from the first anchor point in a posterior direction. As such, the extension limiting strap extends between the first and second anchor points and the anchoring interval is at least about one quarter the circumference of the waist belt.

The present teachings will be further understood from the drawings and the following detailed description.

### BRIEF DESCRIPTION OF THE DRAWINGS

FIG. 1 is a front view of a hip brace mounted on the body of a wearer.
FIG. 2 is a rear view of a hip brace of FIG. 1.
FIG. 3 is a partial plan view of the hip brace of FIG.1 laid out flat to show its outer surface.
FIG. 4 is a rear plan view of a waist belt having utility in the hip brace of FIG. 1.
FIG. 5 is a plan view of a waist cuff having utility in the hip brace of FIG. 1.

### DETAILED DESCRIPTION

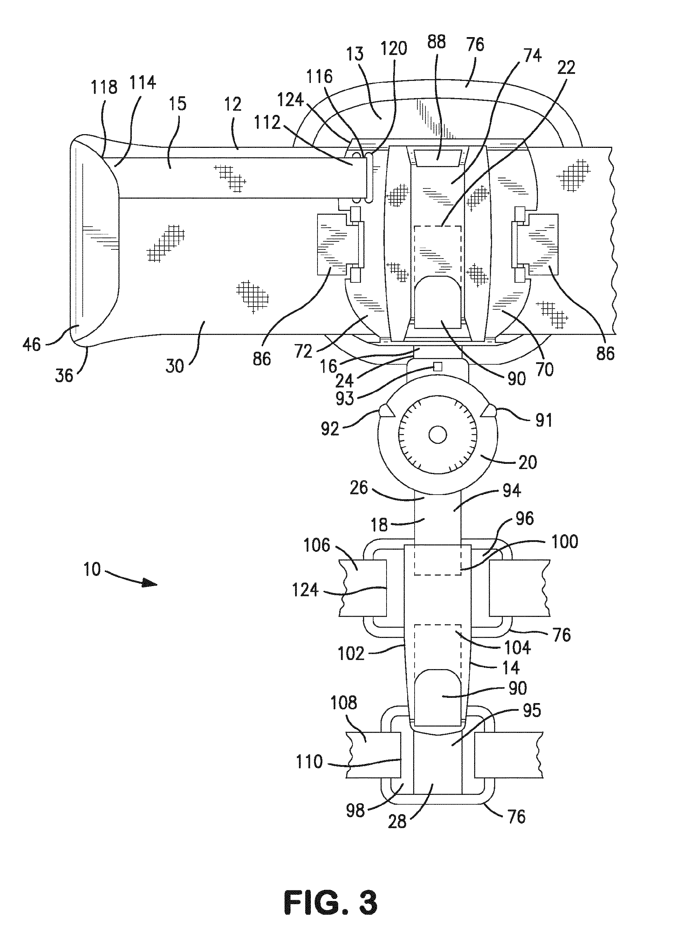

Referring to FIGS. 1-5, a hip brace is shown and generally designated 10. The hip brace 10 comprises a waist belt 12, a waist cuff 13, a longitudinal support assembly 14, and a hip joint extension limiting strap 15. The longitudinal support assembly 14 includes a first or upper support member 16, a second or lower support member 18 and a hinge 20. The waist belt 12 is worn around the waist of a wearer having an affected hip joint and functions in combination with the waist cuff 13 as a first or upper anchor for the longitudinal support assembly 14. The waist belt 12 may also function in an ancillary role as a circumferential support for the lumbar region of the wearer.

As a general rule, the hip brace 10 is configured so that the waist cuff 13 and longitudinal support assembly 14 are positioned on the same side of the body of the wearer as the affected hip joint to support and stabilize the affected hip joint. In the present example case shown in the drawings and described below, the affected hip joint is on the right side of the body. Accordingly, the hip brace 10 is configured so that the waist cuff 13 and longitudinal support assembly 14 are also positioned on the right side of the body. However, it is apparent to one of ordinary skill in the art that if the affected hip joint is on the left side of the body, the hinge brace 10 of the present teachings is readily reconfigurable so that the waist cuff 13 and longitudinal support assembly 14 can be correspondingly positioned on the left side of the body. This alternate configuration is likewise within the scope of the present disclosure.

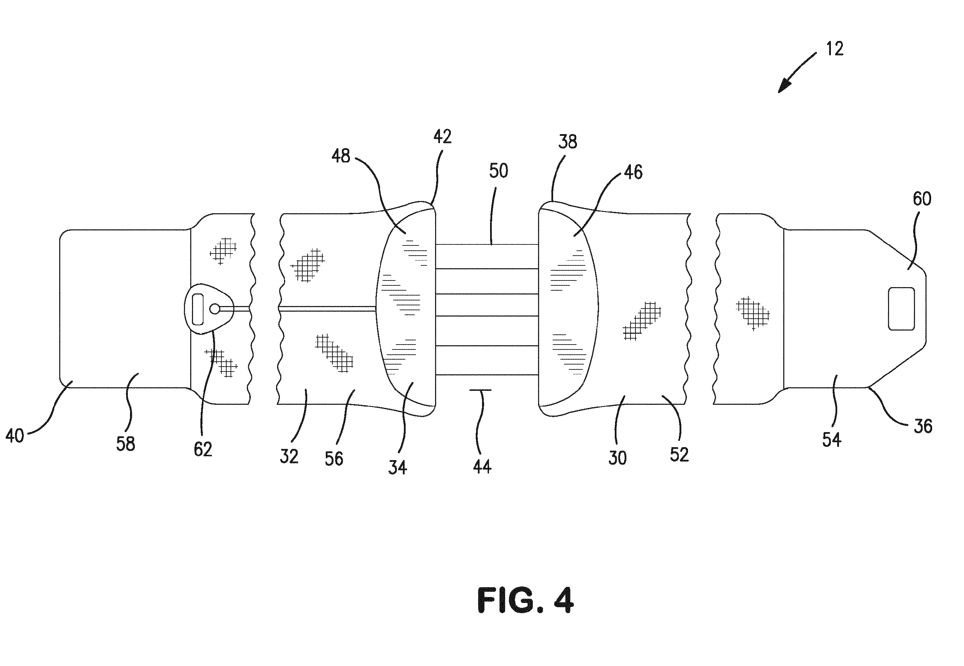

With specific reference to FIG. 4 and continuing reference to the remaining FIGS., the waist cuff 13 engages and attaches to the waist belt 12 at a waist position on the right lateral side of the waist belt 12 which is immediately above the affected hip and which longitudinally aligns with the affected hip. Specific details of the manner of attachment are described hereafter. The first support member 16 has a first or upper end 22 which engages and attaches to the waist cuff 13. The first support member 16 extends downward from waist cuff 13 in alignment with the longitudinal axis of the wearer's torso until it reaches a hip position adjacent to the affected hip joint. The hinge 20 is likewise positioned at the hip position adjacent to the hip joint and the first support member 16 has a second or lower end 24 which attaches to the hinge 20. The second support member 18 has a first or upper end 26 which also attaches to the hinge 20 such that the hinge 20 rotatably connects the first and second support members 16, 18 to one another enabling rotational displacement of each relative to the other. The second end 24 of the first support member 16 and the first end 26 of the second support member 18 are preferably bent or bowed slightly outward so that the hinge 20, which is typically thicker than the first or second support member 16, 18, does not unduly impinge against the hip joint. The second support member 18 extends downward from the hinge 20 along the right lateral side of the wearer's thigh until terminating at a thigh position adjacent to a point on the thigh above the knee. The terminating end of the second support member 18 is its second or lower end 28.

The waist belt for the hip brace of the present disclosure is not limited to any one specific configuration or construction. However, a preferred embodiment of a waist belt having utility in the hip brace of the present teachings is selected from a type of orthopedic supports generally known as lumbar supports or lumbar braces. A specific embodiment of a lumbar support which has utility as the waist belt 12 for the hip brace 10 of the present teachings is described hereafter for purposes of illustration. It is, nevertheless, understood that other lumbar supports or belt-like structures may alternatively have utility as a waist belt for the present hip brace.

The waist belt 12 is a lumbar support which resembles a conventional weightlifting belt and is configured to be worn around the waist and lumbar region of the wearer. The waist belt 12 comprises a first belt segment 30, a separate second belt segment 32 and a mechanical advantage tensioning device 34. The first belt segment 30 has two ends, namely, an attachment end 36 and an adjustment end 38, and the second belt segment 32 similarly has an attachment end 40 and an adjustment end 42. The adjustment ends 38, 42 of the first and second belt segments 30, 32, respectively, are positioned adjacent to one another on the waist belt 12, but preferably do not engage one another, thereby creating a discontinuity which defines a gap 44 between them.

The mechanical advantage tensioning device 34 includes a first housing 46 and a second housing 48. The first and second housings 46, 48 are positioned on either side of the gap 44, respectively, in the assembled waist belt 12. In particular, the first housing 46 is mounted on the first belt segment 30 proximal to the adjustment end 38 thereof and the second housing 48 is mounted on the second belt segment 32 proximal to the adjustment end 42 thereof. As such, the first and second belt segments 30, 32 function as an effective support base for the first and second housings 46, 48 respectively. Mounting of the housings 46, 48 on the belt segments 30, 32, respectively, may be effected by substantially permanent attachment of the housings 46, 48 to the belt segments 30, 32 using conventional permanent attachment means such as riveting, gluing, welding, sewing, stapling, screwing, or the like. Alternatively, mounting may be effected by selective releasable attachment using conventional releasable attachment means such as hook and loop fasteners (sold under the trade name VELCRO) or the like.

The mechanical advantage tensioning device 34 additionally includes a tensioning line 50. The tensioning line 50 is preferably a relatively thin (i.e., small diameter), light-weight, highly-pliant, high-strength, wear-resistant, and friction-resistant monofilament or multi-filament line. In the case of a multi-filament line, the filaments may be woven, braided or twisted together. The tensioning line 50 is also preferably relatively non-stretchable. Lines satisfying the above criteria and having utility herein are commonly considered as including cords, strings, laces, threads, wires or the like. Specific examples include lines which are constructed similar to conventional laces for recreational footwear or conventional drawstrings for window mini-blinds.

The first belt segment 30 and the second belt segment 32 are both fabricated from an at least somewhat pliant material such as cloth, laminate, solid foam, leather, or the like, which is preferably less pliant than the tensioning line 50. In any case, the material of the belt segments 30, 32 is preferably in essentially non-stretchable, at least in the circular direction extending around the circumference of the waist of the wearer. In the present embodiment, the first belt segment 30 has a posterior section 52 formed from a first belt material and an anterior section 54 continuous with the posterior section 52 which is formed from a second belt material. The first belt material is preferably a cloth/foam/cloth laminate and the second belt material is preferably a unitary pliant cloth. The cloth of both the first and second belt materials preferably has a nappy surface which can function as a loop component of a selectively releasable hook and loop fastener. As such, a corresponding hook component of a hook and loop fastener can be releasably attached to any point across essentially the entire inner or outer face of the first belt segment 30. In addition, the added foam layer of the first belt material renders the posterior section 52 overall thicker and less pliant (i.e., stiffer) than the anterior section 54 of the first belt segment 30. The posterior section 52 is also preferably configured with a wider footprint than the anterior section 54.

The second belt segment 32 likewise has a posterior section 56 and an anterior section 58 which are constructed in essentially the same manner as the described above with respect to the first belt segment 30. As a result of this configuration, the present embodiment of the waist belt 12 advantageously provides more support and less rearward flexibility to the lumbar region of the wearer while providing less support and more forward flexibility to the abdominal region of the wearer. Although not shown, the first and second belt segments 30, 32, and particularly the thicker, less breathable posterior sections 52, 56 thereof, may have a plurality of small openings formed therethrough for ventilation. The thicker posterior sections 52, 56 may also have a plurality of spaced-apart grooves (not shown) formed therein. The grooves are aligned in correspondence with the longitudinal axis of the wearer's body to provide the stiffened posterior sections 52, 56 with an articulate construction which advantageously facilitates conformance of the waist belt 12 to the arcuate contours of the wearer's body. It is also within the scope of the present disclosure to integrate one or more rigid reinforcing elements (not shown) such as plates, stays or the like formed from plastics, metals, resins, composites or the like into the waist belt 12 and more particularly into the first and/or second belt segments 30, 32 in a manner well known to one of ordinary skill in the art. The reinforcing elements can also be externally attached to the waist belt 12 as desired. In any case, any optional reinforcing elements added to the waist belt 12 preferably enhance the support function thereof. The term "rigid", unless used as a relative term, refers to elements or materials which are highly resistant to deformation, but which may or may not undergo elastic deformation in response to a significant force.

Each belt segment 30, 32 has a substantially similar configuration to the other which resembles a half-length of a widened belt that has been bisected along its posterior centerline. The first and second belt segments 30, 32 are preferably sized such that when their adjustment ends 38, 42 are posteriorly connected by the mechanical advantage tensioning device 34, the first and second belt segments 30, 32 and mechanical advantage tensioning device 34 in combination fully encircle the waist of the wearer. This enables the user to cinch the waist belt 12 on the body of the wearer in the following manner. The user grasps the attachment ends 36, 40 of the first and second belt segments 30, 32 respectively, and posteriorly positions the adjustment ends 38, 42 against the lower back of the wearer adjacent to the spine, but spaced apart from one another. The gap 44 between the adjustment ends 38, 42 is bridged by the mechanical advantage tensioning device 34 which connects the adjustment ends 38, 42 to one another, even while the mechanical advantage tensioning device 34 preferably remains in a relaxed state.

The user manually wraps the length of the second belt segment 32 anteriorly around one side of the wearer's waist and pulls the attachment end 40 tight, anteriorly positioning it over the wearer's abdomen. The user likewise manually wraps the length of the first belt segment 30 anteriorly around the other side of the wearer's waist and pulls the attachment end 36 tight, anteriorly positioning it over the wearer's abdomen in overlapping relation to the attachment end 40 of the second belt segment 32. A releasable fastening tab 60 is integral with the overlapping attachment end 36 of the first belt segment 30 which has hook component of a hook and loop fastener on its inner face. The user releasably fastens the attachment end 36 of the first belt segment 30 to the loop component on the outer face of the second belt segment 32 described above which the attachment end 40 overlaps, thereby cinching the waist belt 12 on the body of the wearer. It is further understood that although a hook and loop fastener is described above as a preferred releasable fastening means for cinching the waist belt 12 on the body, other conventional releasable fasteners have utility herein such as buckles, zippers, buttons, laces and the like and all fall within the scope of the present disclosure.

Once the waist belt is cinched on the body of the wearer, the waist belt 12 is preferably further adjustably tensioned by means of the mechanical advantage tensioning device 34 before the wearer secures the longitudinal support assembly 14. The tensioning line 50 has two internal ends (not shown) which attach internally to the housings 46, 48. The path of the tensioning line 50 generally extends from each of its internal ends back and forth between the two housings 46, 48 as well as within the interiors of the two housings 46, 48. The midsection of the tensioning line 50 exits the interior of the second housing 48 on the lateral side thereof and loops through a tensioning handle 62. The tensioning line 50 is tensioned by pulling the tensioning handle 62 laterally, which draws the tensioning line 50 further out of the interior of the second housing 48. Conversely, the tensioning line 50 is relaxed by releasing the tensioning handle 62 and allowing the tensioning line 50 to be drawn back into the interior of the second housing 48. Tensioning the tensioning line 50 draws the opposing first and second housings 46, 48 closer together, thereby drawing the adjustment ends 38, 42 of the underlying waist belt 12 closer together and causing the waist belt 12 to fit more snugly around the waist. Additional details of the construction and operation of the mechanical advantage tensioning device 34 are well known to one of ordinary skill in the art. An example mechanical advantage tensioning device having utility with the waist belt in the hip brace of the present teachings is shown and described in detail in co-owned, co-pending U.S. Patent Application No. 13/831,646, entitled "Anti-Twist Mechanism for a Mechanical Advantage Tensioning Device on an Orthosis", filed on March 15, 2013, which is incorporated herein by reference.

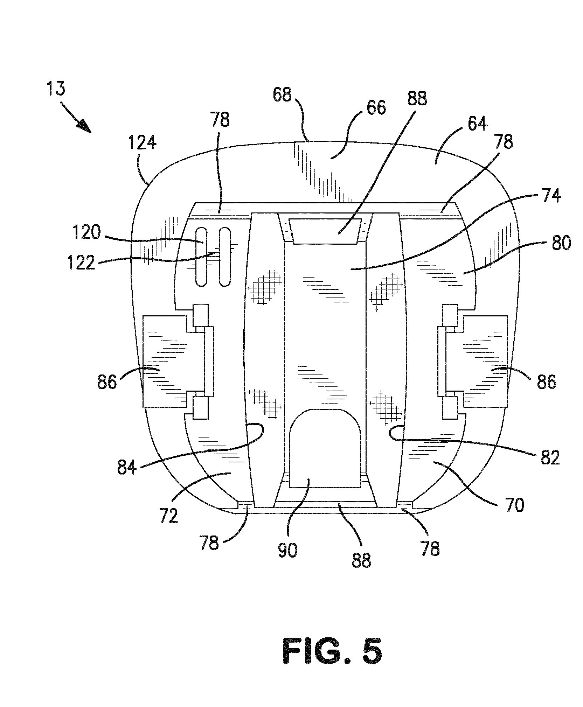

With specific reference to FIG. 5 and continuing reference to the remaining FIGS., the waist cuff 13 of the hip brace 10 is preferably a unitary structure having a main body 64 with an outer surface 66 and an inner surface 68. A first or anterior waist belt guide 70, second or posterior waist belt guide 72 and a first support member housing 74 are preferably integrally formed with the main body 64 on the outer surface 66 thereof. The main body 64 has a broad plate-like structure and the inner surface 68 is configured to continuously engage a broad area of the wearer's torso at the waist on the same lateral side as the affected hip joint. As such, the inner surface 68 of the main body 64 has an arcuate contour generally conforming to the curved contour on the side of the waist. As recited above, the waist cuff 13 functions in combination with the waist belt 12 as the first anchor for the longitudinal support assembly 14. In the performance of this function, the main body 64 transfers force loads from the hinge 20 to the waist belt 12. When the wearer of the hinge brace 10 attempts to flex or extend the hip joint beyond flexion or extension limits set for the hinge 20 in a manner described below, the longitudinal support assembly 14 applies a moment of force to the waist cuff 13 which the main body 64 transfers to the waist belt 13. This dynamic moment of force is resisted by the generally static counter-force of the waist belt 12 and the wearer's body, thereby resisting undesirable rotational or linear displacement of the waist cuff 13 relative to the wearer's torso.

During flexion, the hip joint is bent by rotating it anteriorly to decrease the angle of the joint, e.g., rotating the thigh anteriorly decreases the angle between the thigh and the torso. The ability of the waist belt 12 and wearer's body to resist the moment of force that the longitudinal support assembly 14 applies to the waist cuff 13 tends to be greater during flexion. As a result, the resistant counter-force of the waist belt 12 and body usually exceeds the moment of force on the waist cuff 13 during flexion and the waist cuff 13 desirably remains essentially static, particularly if the waist belt 12 is worn around the waist with sufficient tension.

During extension, the hip joint is straightened by rotating it posteriorly to increase the angle of the joint, e.g., rotating the thigh posteriorly increases the angle between the thigh and the torso. However, the ability of the waist belt 12 and wearer's body to resist the moment of force that the longitudinal support assembly 14 applies to the waist cuff 13 tends to be less during extension, particularly when there is a normal or excessive build-up of softer tissue around the lateral and posterior sides of the waist where the waist cuff 13 and waist belt 12 are positioned. This softer tissue provides less static resistance to the moment of force on the waist cuff 13 during extension. As a result, the moment of force often exceeds the resistant counter-force during extension, thereby rendering the waist cuff 13 more susceptible to undesirable rotational or linear displacement relative to the wearer's torso. The extension limiting strap 15, which is described in more detail below, is designed to mitigate the effect of the moment of force on the waist cuff 13 during extension of the hip joint.

In any case, the main body 64 and the remaining elements of the waist cuff 13 recited above, whether integrally formed in a unitary construction or not, are preferably constructed formed from one or more rigid materials, such as plastics, metals, resins, composites or the like, which are substantially less pliant than the pliant material of the waist belt 12. Due to the rigidity of the waist cuff 13, its inner surface 68 is preferably provided with a pad 76 which intervenes between the waist cuff 12 and body of the wearer to cushion the wearer from the relatively hard inner surface 68 of the waist cuff 12 when the hip brace 10 is mounted on the body. The pad 76 is formed from a relatively soft material such as a foam or the like and is preferably releasably attached to the inner surface 68 of the waist cuff 12 by means of a hook and loop fastener or the like (not shown).

The first and second waist belt guides 70, 72 are essentially identical elongate elements longitudinally aligned with the longitudinal axis of the torso. The first and second waist belt guides 70, 72 are positioned on the outer surface 66 of the main body 64 on opposite sides of the first support member housing 74. Each waist belt guide 70, 72 has a three-sided configuration with a stubbed leg 78 on each of its opposite ends which are attached to and extend a short distance away from the outer surface 66 of the main body 64. A cross member 80 extends between and connects the legs 78 of the waist belt guide 70, 72 , thereby defining an open first waist belt slot 82 and an open second waist belt slot 84. The first waist belt slot 82 is between the outer surface 66 of the main body 64 and the inner surface of the first waist belt guide 70 and the second waist belt slot 84 is between the outer surface 66 of the main body and the inner surface of the second waist belt guide 72. The first support member housing 74 is also an elongate element longitudinally aligned with the longitudinal axis of the torso. The first support member housing 74 is centrally positioned on the outer surface 66 of the main body 64 between the first and second waist belt guides 70, 72. The first support member housing 74 is likewise configured with a stubbed leg on each end to raise it above the outer surface 66 of the main body 64 in a similar manner as the first and second waist belt guides 70, 72. Raising the first support member housing 74 provides the waist belt 12 with sufficient clearance to pass underneath the first support member housing 74 between the outer surface 66 of the main body 64 and the inner surface of the first support member housing 74.

The waist cuff 13 attaches to the waist belt 12 by slidably threading the attachment end 36 of the first belt segment 30 through the first waist belt slot 82, passing the end 36 under the first support member housing 74 and slidably threading the end 36 through the second waist belt slot 84. The first and second waist belt guides 70, 72 each additionally include a belt lock 86 which is a flap rotatably attached to the waist belt guide 70, 72. The belt lock 86 has a hook component of a hook and loop fastener on its inner face which enables the belt lock 86 to releasably lock the waist belt 12 into place within the first and second waist belt slots 82, 84 when the belt lock 86 is rotated down onto the loop component on the outer surface of the waist belt 12. The belt locks 86 are similar to those shown and described in U.S. Patent No. 8,277,403, which is incorporated herein by reference.

The first support member housing 74 is constructed with a longitudinal channel 88 formed through its interior which is shaped and sized to slidably receive the first end 22 of the first support member 16 therein. The length of the first support member 16 is preferably adjustable by slidably telescoping the first support member 16 in or out of the first support member housing 74 to shorten or lengthen the first support member 16, respectively. The first support member housing 74 includes a support member lock 90 which is rotatably attached to the first support member housing 74. The support member lock 90 releasably locks the first support member 16 into place within the channel 88 at a desired length when the support member lock 90 is rotated downward to impose a pressing force against the surface of the first support member 16. The first support member 16 is unlocked for readjusting its length by rotating the support member lock 90 upward to release the pressing force against the surface of the first support member 16. The first support member housing 74 and lock 90 are similar to those shown and described in U.S. Patent No. 8,277,403, which is incorporated above by reference.

The hinge 12 is preferably a releasably locking rotational hinge with adjustable rotation limits. As such, a preferred hinge 12 includes an adjustable flexion rotation stop 91, an adjustable extension rotation stop 92 and a releasable hinge lock 93. The adjustable flexion rotation stop 91 enables the user to limit the range of hip joint flexion by setting the flexion rotation stop 91 at a desired flexion angle limit. A typical range of hip joint angles within which the user can selectively set the flexion rotation stop 91 to a flexion angle limit is between 60° and 180°. It is noted that the hip joint is at a neutral position when the hip joint angle is 180°, e.g., when the subject is in a fully standing or fully prone position. The hip joint angle is about 90° when the subject is in a normal seated position. The adjustable extension rotation stop 92 similarly enables the user to limit the range of hip joint extension by setting the extension rotation stop 92 at a desired extension angle limit. A typical range of hip joint angles within which the user can selectively set the extension rotation stop 92 to an extension angle limit is between 110° and 190°. The releasable hinge lock 93 enables the user to releasably fix the hip joint at a single hip joint angle by selectively manually activating the hinge lock 93. Details of an example hinge having utility in the hip brace of the present teachings are disclosed in U.S. Patent No. 7,235,059, which is incorporated herein by reference. Notwithstanding the above, it is understood that the present hinge is not limited to any one specific construction. Thus, many conventional hinges for orthopedic braces, which enable rotation of the first and second support members about the hinge, are alternatively employed as the hinge of the present hip brace within the scope of the present disclosure.

The second support member 18 which extends downward from the hinge 20 along the right lateral side of the wearer's thigh is divided into two segments, i.e., a first or upper support segment 94 and a second or lower support segment 95. The second support member 18 is preferably secured to the wearer's thigh by a thigh strapping system which functions as a second or lower anchor for the longitudinal support assembly 14. A preferred thigh strapping system includes a first or upper strap retainer 96 and a second or lower strap retainer 98. The inner surface of each strap retainer 96, 98 is preferably provided with a pad 76 of the type described above which intervenes between the strap retainer 96, 98 and body of the wearer to cushion the wearer from the relatively hard inner surface of the strap retainer 96, 98. The first support segment 94 of the second support member 18 has a first or upper end which is the same as the first end 26 of the second support member 18 and attaches to the hinge 20. The first support segment 94 also has a second or lower end 100 which is fixably attached to the first strap retainer 96.

The first strap retainer 96 includes a second support member housing 102 which has a substantially similar construction to the first support member housing 74. As such, the second support member housing 102 also has essentially the same longitudinal channel 88 formed through its interior. The second support segment 95 of the second support member 18 has a first or upper end 104 and the channel 88 of the second support member housing 102 is shaped and sized to slidably receive the first end 104 therein. Accordingly, the first strap retainer 96 connects the first and second support segments 94, 95. The length of the second support segment 95 is preferably adjustable in the same manner as described above with respect to the first support member 16. The second support member housing 102 also has essentially the same support member lock 90 as the first support member housing 74. The second support segment 95 of the second support member 18 has a second or lower end which is the same as the second end 28 of the second support member 18 and is fixably attached to the second strap retainer 98.

The thigh strapping system further comprises first and second thigh straps 106, 108. The first thigh strap 106 is threaded through a strap slot 110 in the first strap retainer 96, wrapped under tension around the thigh and retained in position by releasable means such as a hook and loop fastener or strap connectors of the type disclosed in U.S. Patent No. 8,277,403. The second thigh strap 108 is likewise threaded through the strap slot 110 in the second strap retainer 98, wrapped under tension around the thigh and retained in position by releasable means which completes the mounting of the hip brace 10 on the body.

The hip brace 10 is provided with the hip joint extension limiting strap 15 to enhance the performance of the hip brace 10 during operation when the wearer is mobile. The extension limiting strap 15 has a first or anterior end 112 and a second or posterior end 114. The first end 112 of the extension limiting strap 15 is preferably attached to the waist cuff 13 at a first or lateral anchor point 116. The second end 114 of the extension limiting strap 15 is preferably attached to the waist belt 12 to the waist cuff 13 at a second or posterior anchor point 118 which is spaced an anchoring interval apart from the first anchor point 116. The anchoring interval is defined as the distance spanned by the extension limiting strap 15 along the circumference of the waist belt 12 from the first anchor point 116 to the second anchor point 118 in the posterior clockwise direction. It is noted that if the hip brace 10 is configured so that the waist cuff 13 and longitudinal support assembly 14 are positioned on the left lateral side, the anchoring interval is defined as the distance spanned by the extension limiting strap 15 from the first anchor point 116 to the second anchor point 118 in the posterior counter-clockwise direction.

The extension limiting strap 15 is preferably fabricated from a pliant material such as cloth, laminate, leather, or the like, which may be the same or similar to the pliant material used in the waist belt 12 and/or the first and second thigh straps 106, 108. In any case, the extension limiting strap 15 is preferably essentially non-stretchable, at least in the longitudinal direction of the strap 15. The outer surface of the extension limiting strap 15 is preferably integrally formed from the loop component of a hook and loop fastener or, if not integrally formed as such, the extension limiting strap 15 is preferably supplemented with a swath of the loop component which continuously extends across essentially the length of the outer surface of the extension limiting strap 15. A patch of the hook component is also preferably affixed to the first end 112 of the extension limiting strap 15 so that the extension limiting strap 15 can be doubled over itself and the first end 112 releasably fastened to the underlying outer surface of the extension limiting strap 15 by means of the hook and loop fastener. Alternatively, the positions of the hook component at the first end 112 of the extension limiting strap 15 and the loop component on the outer surface of the extension limiting strap 15 can be reversed within the scope of the present disclosure.

In accordance with the present embodiment, attachment of the second end 114 of the extension limiting strap 15 to the waist belt 12 at the second anchor point 118 is effected by substantially permanent attachment means. In particular, the second end 114 of the extension limiting strap 15 is positioned between the adjustment end 38 of the first belt segment 30 and the first housing 46 of the mechanical advantage tensioning device 34. The adjustment end 38, second end 114 and first housing 46 are all essentially permanently fastened to one another. Example permanent fastening means include riveting, gluing, welding, sewing, stapling, screwing, or the like. Attachment of the first end 112 of the extension limiting strap 15 to the waist cuff 13 at the first anchor point 116 is effected by releasable attachment means. In particular, the first end 112 of the extension limiting strap 15 is threaded through one side of a bifurcated strap retention opening 120 in the second waist belt guide 72. The first end 112 is looped around a post 122 which bisects the opening 120 and threaded back through the other side of the strap retention opening 120. The first end 112 is doubled over the outer surface of the strap 15, thereby tensioning the strap 15, and the extension limiting strap 15 is releasably fastened to itself by the hook and loop fastener provided on the strap 15. As such, the anchoring interval, i.e., the distance the extension limiting strap 15 extends clockwise and posteriorly around the circumference of the waist belt 12 is approximately one quarter the circumference of the waist belt 12.

As noted above, a function of the waist cuff 13 is to transfer loads from the hinge 20 to the waist belt 12. In the absence of the extension limiting strap 15, the bulk of the moment of force generated by the longitudinal support assembly 14 during extension of the hip joint is applied to a single point on the waist cuff 13 at its posterior upper (superior) corner 124. Although much of this force is transferred to and distributed across a length of the waist belt 12, the resistive force of the tensioned waist belt 12 and body underlying the waist belt 12 is insufficient to overcome all of the moment of force, particularly when the hip joint is at extension. Consequently, the excessive moment of force that the longitudinal support assembly 14 applies to the waist cuff 13 during extension undesirably displaces the posterior upper corner 124 of the waist cuff 13 forward. In most cases the forward displacement is rotational rather than linear with an anterior direction being a component of the rotational displacement.

It is most preferred to position the first anchor point 116 for the first end 112 of the extension limiting strap 15 at a posterior point on the waist cuff 13 in accordance with the teaching of the present disclosure. However, the position of the second anchor point 118 for the second end 114 of the extension limiting strap 15 shown in the present embodiment is only one example of multiple preferred positions of the second anchor point 118 and resulting anchoring intervals which are within the scope of the present disclosure. Although the present position of the second anchor point is believed optimal under many conditions, it may be desirable to reposition the second anchor point 118 to different location for improved performance under certain conditions. For example, the practitioner could reposition the second anchor point 118 on the opposite side of the gap 44 from the position of the present embodiment and permanently attach the second end 114 of the extension limiting strap 15 to the second housing 48 and underlying second belt segment 32. Alternatively, the second anchor point 118 could be repositioned even further clockwise and posterior from the position of the present embodiment to a contra-lateral point on the waist belt 12 opposite the first anchoring point 116. As such, the anchoring interval would approximate half the circumference of the waist belt 12. Selection of an alternate second anchor point position and correspondingly an alternate anchoring interval is within the purview of the one of ordinary skill in the art following the teaching herein. For example, the practitioner can determine the optimal position for the second anchor point 118 under certain conditions by simple trial and error, observing which anchor point essentially prevents any rotational displacement of the waist cuff 13 during extension of the hip joint under given conditions and selecting that point as the optimal second anchor point 118.

The present embodiment of the hip brace 10 employs permanent attachment means for securing the second end 114 of the extension limiting strap 15 to the second anchor point 118 and releasable attachment means for securing the first end 112 of the extension limiting strap 15 to the first anchor point 116. The first anchor point 116 is chosen as the position for a releasable attachment means because the first anchor point 116 is more anterior than the second anchor point 118 and, therefore, more accessible to the wearer should the wearer desire to adjust the tension of the extension limiting strap 15 when it is in a relaxed state. However, it is within the scope of the present disclosure to reverse this configuration by permanently attaching the first end 112 of the extension limiting strap 15 to the first anchor point 116 and releasably attaching the second end 114 of the extension limiting strap 15 to the second anchor point 118. Alternatively, both ends 112, 114 can be releasably attached to their respective anchor points 116, 118. In addition, the practitioner can provide the user with multiple positions to select as a second anchoring point. For example, the practitioner can permanently attached strap rings to the outer surface of the waist belt 12 at spaced apart intervals posteriorly around its circumference. The user could then releasably attach the second end 114 of the extension limiting strap 15 through a selected strap ring in a conventional manner, thereby selecting the position of the second anchoring point 118.

In any case, the presence of the extension limiting strap 15 on the hip brace 10 augments the ability of the waist cuff 13 to resist rotational displacement during extension of the hip joint by distributing the moment of force away from the posterior upper corner 124 on the waist cuff 13 to a more posterior point on the waist belt 12 further away from the waist cuff 13. It is noted that the extension limiting strap 15 is in a relaxed untensioned state when the hip joint travels to a flexion angle and has no impact on the operation of the hip brace 10. However, when the hip joint travels to an extension angle equal to the extension angle limit that the user has set the hinge 10 to, e.g., 180°, the extension limiting strap 15 transitions to a tensioned state as the moment of force that the longitudinal support assembly 14 applies to the waist cuff 13 urges the waist cuff 13 anteriorly away from the lateral side of the waist belt 12 and pulls the extension limiting strap 15 taut. The tension on the extension limiting strap 15 applies a counter-force to the waist cuff 13 in a posterior direction opposite the direction of the moment of force which prevents the waist cuff 13 from being anteriorly displaced on the wearer's waist. The increased restriction on displacement of the waist cuff 13 during hip joint extension provided by the extension limiting strap 15 of the present teachings ensures that the wearer is unable to extend the hip joint beyond the extension angle limit of the hip brace 10 which the user sets at the hinge 20. For example, if the user sets the extension angle limit to 180° at the hinge 20, the wearer will be unable to extend the hip joint to an extension angle of 190° or, for that matter, any extension angle greater than 180°. In sum, the extension limiting strap 15 enhances control of the wearer's hip joint in extension by connecting the posterior side of the waist cuff 13 to a point further around the posterior side of the waist belt 12.

Therefore, from one viewpoint, there has been described a hip brace having a waist belt, waist cuff, longitudinal support assembly and hip joint extension limiting strap. The waist belt is secured around the waist of a wearer and the waist cuff and longitudinal support assembly are positioned on the side of the body adjacent to the affected hip joint. The waist cuff is attached to the waist belt above the hip joint and the longitudinal support assembly extends from the waist cuff past the hip joint along the length of the thigh and is hinged in correspondence with the hip joint. The extension limiting strap extends posteriorly from a lateral anchor position on the waist cuff to a posterior anchor position on the waist belt and prevents undesirable migration of the waist cuff during hip joint extension.

Further examples of feature combinations included within the presen teachings are set out in the following clauses:
Clause 1. A hip brace comprising: a waist belt wrappable around a waist of a wearer of said hip brace, said waist belt having a circumference including a first lateral side, a second lateral side, a posterior side and an anterior side; a waist cuff having an anterior side and a posterior side, wherein said waist cuff is attached to said first lateral side of said waist belt and said waist cuff is substantially less pliant than said waist belt; a longitudinal support assembly attached to said waist cuff and extending downward away from said waist cuff, wherein said longitudinal support assembly includes a first support member having a first end attached to said waist cuff and a second end, a rotational hinge attached to said second end of said first support member and aligned with a hip joint of the wearer adjacent to said first lateral side of said waist belt, and a second support member having a first end, wherein said hinge is attached to said first end of said second member, thereby rotatably connecting said first and second members; and an extension limiting strap attached to said waist cuff at a first anchor point, attached to said waist belt at a second anchor point positioned an anchoring interval away from said first anchor point in a posterior direction and extending between said first and second anchor points, wherein said extension limiting strap relaxes when said hinge travels in a hip flexion direction and tensions when said hinge travels in a hip extension direction to apply a resistant counter-force to said waist cuff in a first force direction in response to a moment of force applied to said waist cuff by said longitudinal support assembly in a second force direction substantially opposite said first force direction.
Clause 2. The hip brace of clause 1, wherein said first anchor point is proximal to a posterior upper corner of said waist cuff.
Clause 3. The hip brace of clause 1 or 2, wherein said anchoring interval is at least about one quarter the circumference of said waist belt.
Clause 4. The hip brace of clause 1, 2 or 3, wherein said waist cuff is rigid.
Clause 5. The hip brace of any preceding clause, wherein said first force direction is anterior and said second force direction is posterior.
Clause 6. The hip brace of any preceding clause, wherein said extension limiting strap is pliant.
Clause 7. The hip brace of any preceding clause, wherein said hinge has an extension rotation stop setable to a hip joint extension angle limit.
Clause 8. The hip brace of clause 7, wherein said hip joint extension angle limit is within a range between about 110° and about 190°.
Clause 9. A hip brace comprising: a waist belt wrappable around a waist of a wearer of said hip brace, said waist belt having a circumference including a first lateral side, a second lateral side, a posterior side and an anterior side; a waist cuff attached to said waist belt, wherein said waist cuff is substantially more rigid than said waist belt; a longitudinal support assembly attached to said waist cuff and extending away from said waist cuff; and an extension limiting strap attached to said waist cuff at a first anchor point, attached to said waist belt at a second anchor point positioned an anchoring interval away from said first anchor point in a posterior direction and extending between said first and second anchor points, wherein said extension limiting strap relaxes when said longitudinal support assembly travels in a hip flexion direction and tensions when said longitudinal support assembly travels in a hip extension direction to apply a resistant counter-force to said waist cuff in a first force direction in response to a moment of force applied to said waist cuff by said longitudinal support assembly in a second force direction substantially opposite said first force direction.
Clause 10. The hip brace of clause 9, wherein said first anchor point is proximal to a posterior upper corner of said waist cuff.
Clause 11. The hip brace of clause 9 or 10, wherein said anchoring interval is at least about one quarter the circumference of said waist belt.
Clause 12. The hip brace of clause 9, 10 or 11, wherein said waist cuff is rigid.
Clause 13. The hip brace of any of clauses 9 to 12, wherein said first force direction is anterior and said second force direction is posterior.
Clause 14. The hip brace of any of clauses 9 to 13, wherein said extension limiting strap is pliant.
Clause 15. A hip brace comprising: a waist belt wrappable around a waist of a wearer of said hip brace, said waist belt having a circumference including a first lateral side, a second lateral side, a posterior side and an anterior side; a waist cuff having an anterior side and a posterior side, wherein said waist cuff is attached to said first lateral side of said waist belt and said waist cuff is substantially more rigid than said waist belt; a longitudinal support assembly attached to said waist cuff and extending downward away from said waist cuff, wherein said longitudinal support assembly includes a first support member having a first end attached to said waist cuff and a second end, a rotational hinge attached to said second end of said first support member and aligned with a hip joint of the wearer adjacent to said first lateral side of said waist belt, and a second support member having a first end, wherein said hinge is attached to said first end of said second member, thereby rotatably connecting said first and second members; and an extension limiting strap attached to said waist cuff at a first anchor point, attached to said waist belt at a second anchor point positioned an anchoring interval away from said first anchor point in a posterior direction and extending between said first and second anchor points, wherein said anchoring interval is at least about one quarter the circumference of said waist belt.
Clause 16. The hip brace of clause 15, wherein said first anchor point is proximal to a posterior upper corner of said waist cuff.
Clause 17. The hip brace of clause 15 or 16, wherein said waist cuff is rigid.
Clause 18. The hip brace of clause 15, 16 or 17, wherein said first force direction is anterior and said second force direction is posterior.
Clause 19. The hip brace of any of clauses 15 to 18, wherein said extension limiting strap is pliant.
Clause 20. The hip brace of any of clauses 15 to 19, wherein said hinge has an extension rotation stop setable to a hip joint extension angle limit.

While the forgoing example embodiments of the present teachings have been described and shown, it is understood that alternatives and modifications, such as those suggested and others, may be made thereto and fall within the scope of the present teachings and of the appended claims.

## Claims

1. A hip brace comprising:
a waist belt wrappable around a waist of a wearer of said hip brace, said waist belt having a circumference including a first lateral side, a second lateral side, a posterior side and an anterior side;
a waist cuff attached to said waist belt, wherein said waist cuff is substantially more rigid than said waist belt;
a longitudinal support assembly attached to said waist cuff and extending away from said waist cuff; and
an extension limiting strap attached to said waist cuff at a first anchor point, attached to said waist belt at a second anchor point positioned an anchoring interval away from said first anchor point in a posterior direction and extending between said first and second anchor points, wherein said extension limiting strap relaxes when said longitudinal support assembly travels in a hip flexion direction and tensions when said longitudinal support assembly travels in a hip extension direction to apply a resistant counter-force to said waist cuff in a first force direction in response to a moment of force applied to said waist cuff by said longitudinal support assembly in a second force direction substantially opposite said first force direction.

2. The hip brace of claim 1, wherein said first anchor point is proximal to a posterior upper corner of said waist cuff.

3. The hip brace of claim 1 or 2, wherein said anchoring interval is at least about one quarter the circumference of said waist belt.

4. The hip brace of claim 1, 2 or 3, wherein said waist cuff is rigid.

5. The hip brace of any of claims 1 to 4, wherein said first force direction is anterior and said second force direction is posterior.

6. The hip brace of any of claims 1 to 5, wherein said extension limiting strap is pliant.

7. The hip brace of any preceding claim, further comprising:
the waist cuff having an anterior side and a posterior side, wherein said waist cuff is attached to said first lateral side of said waist belt and said waist cuff is substantially less pliant than said waist belt;
the longitudinal support assembly including a first support member having a first end attached to said waist cuff and a second end, a rotational hinge attached to said second end of said first support member and aligned with a hip joint of the wearer adjacent to said first lateral side of said waist belt, and a second support member having a first end, wherein said hinge is attached to said first end of said second member, thereby rotatably connecting said first and second members; and
wherein said extension limiting strap relaxes when said hinge of said longitudinal support assembly travels in a hip flexion direction and tensions when said hinge travels in a hip extension direction to apply the resistant counter-force to said waist cuff.

8. The hip brace preceding 7, wherein said hinge has an extension rotation stop setable to a hip joint extension angle limit.

9. The hip brace of claim 8, wherein said hip joint extension angle limit is within a range between about 110° and about 190°.

10. A hip brace comprising:
a waist belt wrappable around a waist of a wearer of said hip brace, said waist belt having a circumference including a first lateral side, a second lateral side, a posterior side and an anterior side;
a waist cuff having an anterior side and a posterior side, wherein said waist cuff is attached to said first lateral side of said waist belt and said waist cuff is substantially more rigid than said waist belt;
a longitudinal support assembly attached to said waist cuff and extending downward away from said waist cuff, wherein said longitudinal support assembly includes a first support member having a first end attached to said waist cuff and a second end, a rotational hinge attached to said second end of said first support member and aligned with a hip joint of the wearer adjacent to said first lateral side of said waist belt, and a second support member having a first end, wherein said hinge is attached to said first end of said second member, thereby rotatably connecting said first and second members; and
an extension limiting strap attached to said waist cuff at a first anchor point, attached to said waist belt at a second anchor point positioned an anchoring interval away from said first anchor point in a posterior direction and extending between said first and second anchor points, wherein said anchoring interval is at least about one quarter the circumference of said waist belt.

11. The hip brace of claim 10, wherein said first anchor point is proximal to a posterior upper corner of said waist cuff.

12. The hip brace of claim 10 or 11, wherein said waist cuff is rigid and/or said extension limiting strap is pliant.

13. The hip brace of claim 10, 11 or 12, wherein said first force direction is anterior and said second force direction is posterior.

14. The hip brace of any of claims 10 to 13, wherein said hinge has an extension rotation stop setable to a hip joint extension angle limit.

15. The hip brace of any of claims 10 to 14, wherein said extension limiting strap relaxes when said longitudinal support assembly travels in a hip flexion direction and tensions when said longitudinal support assembly travels in a hip extension direction to apply a resistant counter-force to said waist cuff in a first force direction in response to a moment of force applied to said waist cuff by said longitudinal support assembly in a second force direction substantially opposite said first force direction.
